# EUROPÄISCHE PATENTSCHRIFT

(11) **EP 0 486 788 B1**
(45) Veröffentlichungstag und Bekanntmachung des Hinweises auf die Patenterteilung: **10.08.1994**
(21) Anmeldenummer: 91116191.7
(22) Anmeldetag: 24.09.1991
(51) Int. Cl.: H01M 2/10, A61F 2/72

(54) **Akkumulator**
Accumulator
Accumulateur

(30) Priorität: 22.11.1990 DE 9015923 U
(43) Veröffentlichungstag der Anmeldung: 27.05.1992
(73) Patentinhaber: Otto Bock Orthopädische Industrie Besitz- und Verwaltungs-Kommanditgesellschaft, 37115 Duderstadt (DE)
(72) Erfinder: Meier, Hans Adolf, 3408 Duderstadt (DE)
(74) Vertreter: Gramm, Werner, Prof., Dipl.-Ing.

(56) Entgegenhaltungen:
- EP-A- 0 356 205

## Beschreibung

Die Erfindung betrifft einen Akkumulator zum Einbau in eine elektrische Fremdkraftprothese, mit stirnseitig eingelassenen Kontaktgabeln zum Eingriff in prothesenseitig vorgesehene schneidenförmige Kontakte.

Eine derartige Ausführungsform läßt sich der deutschen Patentschrift Nr. 2 542 933 entnehmen. Für elektrische Fremdkraftprothesen gibt es myoelektrische Steuerungen sowie elektromechanische Steuersysteme, die von einem in die Prothese eingebauten Akkumulator angetrieben werden. Der vorbekannte, wiederaufladbare Akkumulator wird in einer Halterung eingesetzt, die sich in alle Schaftformen integrieren läßt und durch die geringen Außenmaße auch ein Einsetzen bei langen Stümpfen ermöglicht. In der Halterung sind schneidenförmig ausgebildete Kontakte vorgesehen, die beim Einlegen des Akkumulators in dessen Kontaktgabeln eingreifen. Zur Festlegung des Akkumulators in seiner Halterung ist ein Schnappverschluß vorgesehen.

Ein Schalter für die elektrische Versorgung ist außerhalb des Akkumulators bzw. seiner Halterung vorgesehen und wird z.B. in einer Handprothese integriert und von außen durch den Handüberzug hindurch betätigt.

Der Erfindung liegt die Aufgabe zugrunde, den vorbekannten Akkumulator hinsichtlich der Handhabung zu vereinfachen.

Diese Aufgabe wird erfindungsgemäß dadurch gelöst, daß auf der den Kontaktgabeln gegenüberliegenden Gehäuseflachseite ein Schiebeschalter angeordnet ist, der quer zur Gehäuselängsachse innerhalb des Gehäuses mit einem Schieber geführt ist, der mit einer Kontaktfeder bestückt und über ein in das Gehäuse federelastisch eingeschnapptes Raststück in seiner Verschiebeebene fixiert ist.

Dabei ist es zweckmäßig, wenn in das Raststück ein mit den Kontaktgabeln bestücktes Anschlußteil lösbar eingeschoben ist, in das eine mit einer Kontaktgabel in elektrischer Verbindung stehende Sicherung eingeschoben ist.

Erfindungsgemäß ist somit der Schalter unmittelbar in den Akkumulator integriert. Dennoch gibt die erfindungsgemäße Konstruktion die Möglichkeit, die Außenabmessungen des Akkumulators gegenüber den herkömmlichen Ausführungsformen zu verringern. Während bei dem herkömmlichen Akkumulator sein Gehäuse aus einem jeweils in Form einer flachen Schale ausgebildeten Ober- und Unterteil besteht, ist es erfindungsgemäß vorteilhaft wenn, Schiebeschalter, Raststück und Anschlußteil in einem Gehäusekopf untergebracht sind, der mit dem Gehäusekörper verklebt ist. Der Schalter kann als Hauptschalter geschaltet sein, mit dem sich die gesamte Stromversorgung zentral ausschalten läßt. Der Akkumulator eignet sich insbesondere zum Einbau in eine Kinderhandprothese, bei der eine Begleitperson des versehrten Kindes einen einfachen Zugriff zu dem Schalter hat.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:

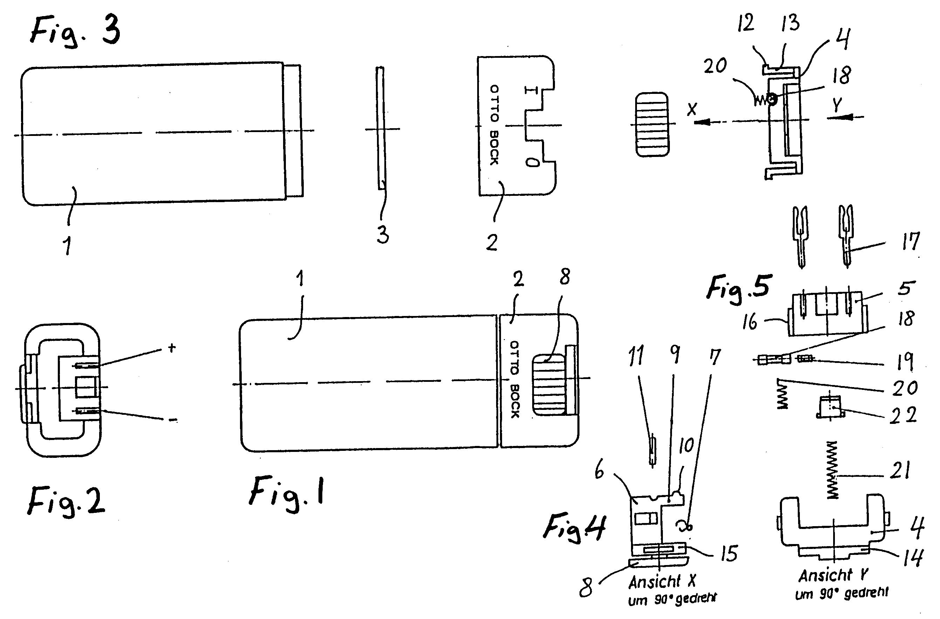

- Figur 1:: einen Akkumulator in Draufsicht;
- Figur 2:: den Akkumulator gemäß Figur 1 in Stirnansicht;
- Figur 3:: den Akkumulator gemäß Figur 1 in Explosionsdarstellung;
- Figur 4:: einen Schiebeschalter in Explosionsdarstellung und
- Figur 5:: in Explosionsdarstellung ein Raststück mit einem Anschlußteil.

Der dargestellte Akkumulator weist ein Gehäuse auf, das sich aus einem Gehäusekörper 1 und einem mit diesem verklebten Gehäusekopf 2 zusammensetzt. In den Gehäusekörper 1 sind ein Distanzkissen 3 sowie ein nicht näher dargestellter Akkublock eingeschoben. Der Gehäusekopf 2 umschließt einen Schiebeschalter, ein Raststück 4 sowie ein Anschlußteil 5, deren Ausbildung nachfolgend beschrieben wird.

Der Schiebeschalter besteht aus einem im Gehäusekopf 2 quer zur Gehäuselängsachse verschiebbar geführten Schieber 6, der mit einer Kontaktfeder 7 bestückt ist und eine Schiebetaste 8 trägt, die auf einer Flachseite des Gehäusekopfes 2 liegt. Der Schieber 6 liegt mit einem federeleastischen Arm 9 an einer nicht näher dargestellten Führung innerhalb des Gehäusekopfes 2 an und greift mit einer am Ende des Armes 9 sitzenden Rastnase 10 in je eine die Schaltstellung des Schiebeschalters fixierende Rast ein. Die Halterung der Kontaktfeder 7 erfolgt über einen Stift 11.

Der Schieber 6 ist in seiner Verschiebeebene innerhalb des Gehäusekopfes 2 über das in dem Gehäuse 2 eingeschobene und hier federelastisch arretierte Raststück 4 fixiert. Das Raststück 4 liegt in eingeschobener Stellung bündig in der Stirnseite des Gehäusekopfes 2 und weist zwei sich gegenüberliegende, mit je einer Rastnase 12 bestückte Federzungen 13 auf, die in der Kupplungsstellung entsprechend ausgebildete Hinterschneidungen des Gehäuseskopfes 2 mit den Rastnasen 12 hintergreifen. Bei stirnseitiger Ansicht des Gehäusekopfes 2 weist das Raststück 4 U-Form auf und übergreift mit einem Steg 14 einen Schenkel 15 des Schiebers 6.

Das Anschlußteil 5 ist zwischen die U-Schenkel des Raststückes 4 und in deren Richtung eingeschoben, wobei seitliche Führungsnasen 16 in entsprechende Führungsnuten auf der Innenseite der genannten U-Schenkel eingreifen. Das Anschlußteil 5 ist auf seiner der Schiebetaste 8 abgewandten Seite mit Kontaktgabeln 17 bestückt, von denen eine mit einer Sicherung 18 elektrisch verbunden ist, die parallel zu dem U-Schenkel des Raststückes 4 in das Anschlußteil 5 eingeschoben und hier von einer Klammer 19 klemmend gehalten ist und mit ihrem freien Ende bei vollständig eingeschobenem Anschlußteil 5 in eine entsprechende Ausnehmung des Raststücks 4 eingreift. Rechtwinklig an der Sicherung 18 greift eine Druckfeder 20 an. Das Anschlußteil 5 hintergreift in seiner montierten Stellung mit den den Kontaktgabeln 17 zugewandten freien Enden seiner Führungsnasen 16 eine nicht näher dargestellte Schulter des Gehäusekopfes 2. Das Anschlußteil 5 muß daher vollständig in das Raststück 4 eingeschoben sein, bevor letzteres seinerseits in die stirnseitige Öffnung des Gehäusekopfes 2 eingeschoben wird.

Zwischen den Kontaktgabeln 17 ist ein gegen die Wirkung einer Feder 21 nach oben verschiebbares Druckteil 22 angeordnet, das beim Einsetzen des Akkumulators in eine angepaßte Halterung innerhalb der Prothese von einer prothesenseitigen Nase eingedrückt wird und bei Entriegelung des Akkumulators diesen einseitig aus seiner Halterung hochdrückt, um so das Herausnehmen des Akkumulators zu erleichtern. Weitere Einzelheiten hierzu können der deutschen Patentschrift 2 542 933 entnommen werden.

## Patentansprüche

1. Akkumulator zum Einbau in eine elektrische Fremdkraftprothese, mit stirnseitig eingelassenen Kontaktgabeln (17) zum Eingriff in prothesenseitig vorgesehene schneidenförmige Kontakte, **dadurch gekennzeichnet**, daß auf der den Kontaktgabeln (17) gegenüberliegenden Gehäuseflachseite ein Schiebeschalter (6,7,8) angeordnet ist, der quer zur Gehäuselängsachse innerhalb des Gehäuses (1,2) mit einem Schieber (6) geführt ist, der mit einer Kontaktfeder (7) bestückt und über ein in das Gehäuse federelastisch eingeschnapptes Raststück (4) in seiner Verschiebeebene fixiert ist.

2. Akkumulator nach Anspruch 1, dadurch gekennzeichnet, daß in das Raststück (4) ein mit den Kontaktgabeln (17) bestücktes Anschlußteil (5) lösbar eingeschoben ist, in das eine mit einer Kontaktgabel (17) in elektrischer Verbindung stehende Sicherung (18) eingeschoben ist.

3. Akkumulator nach Anspruch 1 oder 2, dadurch gekennzeichnet, daß der Schieber (6) mit einem federelastischen Arm (9) an einer Führung anliegt und in diese mit einer Rastnase (12) in je eine die Schaltstellung des Schiebeschalters (6,7,8) fixierende Rast eingreift.

4. Akkumulator nach Anspruch 1, 2 oder 3, dadurch gekennzeichnet, daß das Raststück (4) mit zwei sich gegenüberliegenden, mit je einer Rastnase (12) bestückten Federzungen (13) in eine stirnseitige Öffnung des Gehäuses (1,2) eingeschoben ist und bündig in deren Oberfläche liegt.

5. Akkumulator nach einem der vorhergehenden Ansprüche, dadurch gekennzeichnet, daß der Einschub des Anschlußteiles (5) in das Raststück (4) quer zu dessen eigener Einschubrichtung in das Gehäuse (1,2) liegt.

6. Akkumulator nach Anspruch 5, dadurch gekennzeichnet, daß das Anschlußteil (5) in montierter Stellung eine Schulter des Gehäuses (1,2) hintergreift.

7. Akkumulator nach einem der Ansprüche 2 bis 6, dadurch gekennzeichnet, daß Schiebeschalter (6,7,8), Raststück (4) und Anschlußteil (5) in einem Gehäusekopf (2) untergebracht sind, der mit dem Gehäusekörper (1) verklebt ist.

## Claims

1. Accumulator for installation in an electrical independently powered prosthesis, having contact prongs (17), which are constructed on the end side, for engaging- in blade-shaped contacts provided on the prosthesis side, characterized in that there is arranged on the housing flat side opposite the contact prongs (17) a slide switch (6, 7, 8) which is guided transversely with respect to the housing longitudinal axis within the housing (1, 2) by means of a slide (6) which is equipped with a contact spring (7) and which is fixed in its plane of displacement by way of a latching piece (4) which is resiliently snapped into the housing.

2. Accumulator according to Claim 1, characterized in that there is detachably inserted into the latching piece (4) a connection part (5) which is equipped with said contact prongs (17) and into which there is inserted a fuse (18) which is electrically connected to one contact prong (17).

3. Accumulator according to Claim 1 or 2, characterized in that the slide (6) bears by means of a resilient arm (9) against a guide and engages therein, by means of a latching lug (12), in a respective latch which fixes the switch position of the slide switch (6, 7, 8).

4. Accumulator according to Claim 1, 2 or 3, characterized in that the latching piece (4) is inserted, by means of two mutually opposing spring tongues (13) which are each equipped with a latching lug (12), into an end-side opening in the housing (1, 2) and lies flush with the surface thereof.

5. Accumulator according to one of the preceding claims, characterized in that the insertion of the connection part (5) into the latching piece (4) is transverse to the latching piece's own direction of insertion into the housing (1, 2).

6. Accumulator according to Claim 5, characterized in that in the mounted position the connection part (5) reaches behind a shoulder of the housing (1, 2).

7. Accumulator according to one of Claims 2 to 6, characterized in that the slide switch (6, 7, 8), the latching piece (4) and the connection part (5) are accommodated in a housing head (2) which is glued together with the housing body (1).

## Revendications

1. Accumulateur, destiné à être monté dans une prothèse à motricité externe électrique, avec des fourches de contact (17) insérées frontalement, destinées à s'engager dans des contacts en tranchant prévus du coté prothèse, caractérisé en ce que, sur la face plate de boîtier située en regard des fourches de contact (17) est disposé un interrupteur coulissant (6, 7, 8) guidé transversalement par rapport à l'axe longitudinal du boîtier, à l'intérieur du boîtier (1, 2), avec un curseur (6) équipé d'un ressort de contact (7) et fixé dans son plan de coulissement par l'intermédiaire d'une pièce d'encliquetage (4) venant s'encliqueter, avec l'élasticité d'un ressort, dans le boîtier.

2. Accumulateur selon la revendication 1, caractérisé en ce que, dans la pièce d'encliquetage (4) est insérée amovible une partie de raccordement (5), équipée des fourches de contact (17) et dans laquelle est insérée une sécurité (18) reliée électriquement à une fourche de contact (17).

3. Accumulateur selon la revendication 1 ou 2, caractérisé en ce que le curseur (6) appuie, par un bras (9) ayant l'élasticité d'un ressort, sur un guidage et s'engage dans celui-ci, par un ergot d'encliquetage (12), dans le cliquet spécifique fixant la position de commutation de l'interrupteur coulissant (6, 7, 8).

4. Accumulateur selon la revendication 1, 2 ou 3, caractérisé en ce que la pièce d'encliquetage (4) est insérée, avec deux languettes élastiques (13) placées à l'opposé, équipées chacune d'un ergot d'encliquetage (12), dans une ouverture frontale du boîtier (1, 2) et se place de façon affleurée par rapport à la surface du boîtier.

5. Accumulateur selon l'une des revendications précédentes, caractérisé en ce que l'enfoncement de la partie de raccordement (5) dans la pièce d'encliquetage (4) est effectué dans une direction transversale par rapport à sa direction d'enfoncement propre dans le boîtier (1, 2).

6. Accumulateur selon la revendication 5, caractérisé en ce que la partie de raccordement (5) saisit par l'arrière, en position montée, un épaulement du boîtier (1, 2).

7. Accumulateur selon l'une des revendications 2 à 6, caractérisé en ce que l'interrupteur coulissant (6, 7, 8), la pièce d'encliquetage (4) et la partie de raccordement (5) sont logés dans une tête de boîtier (2) collée sur le corps de boîtier (1).
